# EUROPEAN PATENT APPLICATION

(11) **EP 1 080 727 A1**
(43) Date of publication of application: **07.03.2001**
(21) Application number: 99600011.3
(22) Date of filing: 31.08.1999
(51) Int. Cl.: A61K 35/78, A61P 1/04

(54) **Use of the extract of Thymus Vulgaris for the preparation of a medicament for the treatment of Ulcerative Colitis and Crohn's disease**

(71) Applicant: Emmanouilidis, Anastasios, 15344 Kato Pallini, Attica (GR)
(72) Inventor: Emmanouilidis, Anastasios, 15344 Kato Pallini, Attica (GR)
(74) Representative: Panidou, Alexandra Michael

(57) **Abstract**

Ulcerative Colitis and Crohn's Disease arc two chronic inflammatory diseases of the large bowel, the former, and of the large and/or small bowel, the latter, which share many similarities. Medical treatment, which is common for both diseases, is based on drugs with antiinflammatory and/or immunosuppressant properties.

Corticosteroids are the most important and efficient drugs under current use, followed by Sulfasalazine, 5-Aminosalicyclic acid, 6-Mercaptopurine, Metronidazole, Cyclosporin and several other drugs.

Treatment with one or more of the mentioned drugs does not cure the diseases, but relieves the symptoms. Recurrences are common when treatment is discontinued or the drugs are tapered. Long term treatment, especially with corticosteroids, is accompanied by side effects. It was noticed that the use of the extract of Thymus Vulgaris relieves the symptoms of Ulcerative Colitis and Crohn's Disease in a considerable percentage of patients, without any side effects. The use of this extract has never been reported in the treatment of Ulcerative Colitis and Crohn's Disease.

## Description

Ulcerative Colitis and Crohn's Disease are two chronic inflammatory diseases of the large bowel, the former, and of the large and/or small bowel, the latter. These two diseases share many similarities and, as a result, in some cases, they cannot be distinguished. They are usually referred under the common term "Idiopathic Inflammatory Bowel Disease" or "Inflammatory Bowel Disease (IBD)".

The two diseases run a lifelong course with remissions and exacerbations while, in some cases, symptoms are continuous. Surgical therapy is required in some cases (colectomy or enterectomy) either because fo persistent symptoms or due to the appearence of complications.

The main symptoms of the two diseases are blood per rectum, diarrhea, abdominal pain, fever, anorexia, weight loss and other symptoms related to complications. Complications can be intestinal, such as perforation, stenosis, fistulae or extraintestinal related to liver, bibe ducts, joints, eyes, skin etc.

The severity of the two diseases varies and their attacks can be mild, moderate or severe. In severe cases, if there is not prompt response to conservative management, surgery is mandatory to save patient's life. The two main prognostic factors which affect the severity of the diseases are age at onset and extent of the bowel involvement. The younger the patient is and the more extensive the involvement of the bowel, the higher are the probabilities for a severe course.

### PHARMACEUTICAL THERAPY (PRIOR ART)

Although Ulcerative Colitis and Crohn's Disease are known for more than a hundred years, the former, and for about sixty years, the latter, they still remain two diseases of unknown etiology. As a result, their medical treatment remains empiric, based on drugs which were proven, by controlled studies, to he effective for the relief of the symptoms.

Given that the two diseases are closely related and sometimes indistinguishable, their medical treatment is based on the same pharmaceutical agents. Differences consist mainly in the selection of different first choice drug or different combinations of drugs or different doses.

Many drugs have been tested for the treatment of IBD and many others are under investigation (Table 1). Most of these drugs aimed at the hypothetic pathogenetic mechanism of the two diseases, while others were used following observations which showed good results in some cases. From the many pharmaceutical substances contained in Table 1, currently are in use only those contained in Table 2. These drugs have been shown by controlled studies to be effective in the relief of symptoms in IBD.

Although studies dealing with the medical treatment of IBD usually suggest certain combinations of drugs and certain doses of them, it must be stressed that there are no rigid or routine medical therapies. The well known fluctuation in the severity of IBD and unpredictable response to drugs as well as the appearence of side effects, require frequent modifications of established treatment strategies in order to obtain good results.

**Ulcerative Colitis**: The controlled studies which evaluated various drugs for the treatment of the disease started during the decade of 1950. The first drugs wich were proven to be effective were corticosteroids and ACTH administered per os or parenterally, the former, and only parenterally, the latter^{1,2}. The effectivenes of the two drugs was similar³ but in clinical practice corticosteroids prevailed while ACTH is used in cases not responding to corticosteroids, although it has been obesrved that the drug is more effective when no corticosteroids have been administered recently⁴. In the decade, it has also been proven that corticosteroids are effective when applied locally under the form of enemas or suppositories, in cases of distal disease (proctitis, rectosigmoiditis)⁵. Early in the decade of 1960, the efficacy of Sulfasalazine was established⁶. Compared to corticosteroids, Sulfasalazine was inferior in the acute phase of the disease⁷ and its main indication was to diminish the probabilities of a recurrence⁸. Sometime later, the drug was tried in the form of enemas with positive results⁹, but this form of the drug was not established and it is not available nowadays.

The existence of cases which do not respond to corticosteroids and/or Sulfasalazine, or the appearence of side effects of these two drugs, led the investigation towards immunosuppressant drugs such as Azathioprin. The conclusions of the pertinent studies, which became available the decade of 1970, were that Azathioprin does not help efficiently during the acute phase of the disease, but the concurrent administration with corticosteroids helps to sustain relief of symptoms and allows the tapering of corticosteroids. Azathioprin also helps in corticosteroid resistant cases^{10,11}.

The latest evolution in the pharmaceutical therapy of IBD is the use of 5-Aminosalicylic acid (5-ASA) which is the active ingredient of Sulfasalazine¹². 5-ASA was tried in the decade of 1980, initially in the form of suppositories and enemas for the treatment of proctitis and rectosigmoiditis and was found equally effective as Sulfasalazine per os and corticosteroid enemas^{13,14}. Following this, it was found that 5-ASA administered per os is as effective as Sulfasalazine, but it is better tolerated and is accompanied by less side effects^{15,16}.

It is evident then that, in clinical practice, the attending physician can select one or more of the mentioned drugs, as well as different forms of the same drug according to the extent of the disease, the severity of the attack, the coexistence of other diseases and the probable contraindications to one or more drugs. As it was mentioned earlier, the number of drugs, their doses as well as the duration of therapy, vary in each case. In cases of mild or moderate severity, where usually the extent of the bowel involvement is short, treatment is local with enemas or suppositories or a combination of local and per os treatment, as is seen in Table 3. Corticosteroids should not he used when relief can be achieved with other drugs but one must not hesitate to use them when the patient's condition is severe enough.

Proctitis is almost always managed with suppositories containing either steroids or 5-ASA. In few cases, there is a demand for per os treatment with Sulfasalazine or 5-ASA. In rectosigmoiditis the usual treatment is with corticosteroids or 5-ASA enemas. Quite often though, there is a demand for per os treatment with Sulfasalazine or 5-ASA and, in few cases, corticosteroids per os are needed. Left colitis and more extensive colitis are managed with oral Sulfasalazine or 5-ASA, in combination with oral corticosteroids, as well as corticosteroid and 5-ASA enemas when there is tenesmus. The dose of corticosteroids, such as Presodon, is between 20 and 40 mg per day, that of Sulfasalazine 2 - 4 gr per day and that of 5-ASA 1,2 - 3 gr per day^{17,18,19}. Four to six weeks are usually required for regression of symptoms and, after that, tapering of the drugs is in most cases feasible. When symptoms do not respond to the former treatment, higher doses of corticosteroids are required. Azathioprine may be added in a dose of 1.5 - 2.5 mg/kg/day, so that corticosteroids may later be tapered and discontinued. One must take, though, into account that the action of Azathioprine is delayed and it is usually evident after a mean interval of two months²⁰.

Given that, in almost all cases, the disease will relapse after various intervals of time, there is need to prevent relapses. Corticosteroids in small doses per os were proven ineffective²¹. On the contrary, prophylactic adminstration of Sulfasalazine was found to be effective ⁸ in a dose of 2 gr/day²². More recently, 5-ASA was evaluated as prophylactic treatment, initially in enemas for rectosigmoiditis and left colitis, and it was found to be effective²³. Following this, it was evaluated in the form of tablets and in a dose of 800 mg - 1.5 gr/day and it was also found effective with results similar to those of Sulfasalazine in all forms of Ulcerative Colitis^{24,25}. Although Sulfasalazine and 5-ASA seem to be equally effective in the long-term prophylactic treatment, the latter seems to be superior due to less side effects, but if one considers costs, 5-ASA is more expensive, a fact which is taken under consideration in a long-term treatment.

The question that arises in regard to long-term prophylactic treatment is how long it should last. The prevailing answer is that it should last indefinitely²⁶ and not for a certain period of time, such as a year²⁷. In everyday practice, though, and specifically in Greece, patients discontinue treatment when they are asymptomatic for several months or continue it, but in lower dose from the suggested one.

**Crohn's Disease**: The medical treatment of Crohn's Disease followed the same evolution with that of Ulcerative Colitis, with the use of the same drugs. Initially, the use of various drugs was based on retrospective or open studies. Controlled studies which followed, either confirmed or not validated these results. The basic multicenter trial which evaluated the use of corticosteroids (Prednisone), Sulfasalazine and Azathioprine in the treatment of Crohn's Disease took place in the USA and its conclusions which became available in 1979²⁸, formed the principles for Crohn's Disease medical therapy. As is seen from Table 4, Prednisone and Sulfasalazine are potent drugs for the disease, while Azathioprine is not. The doses of the drugs were higher compared to those used in Ulcerative Colitis, that is 40 - 60 mg of Prednisone, 4 - 6 gr of Sulfasalazine and 2 - 5 mg/kg/day of Azathioprine. The same USA trial also concluded that the concurrent administration of Prednisone and Sulfasalazine was not superior to the administration of only Prednisone and that Sulfasalazine administration does not allow decrease of the dose of Prednisone²⁹. Five years later, the results of a European multicenter trial became available. This trial evaluated the efficacy of 6-Methylprednisolone in a dose equivalent to 60 mg of Prednisone, of Sulfasalazine in a dose of 3 gr/day and of the combination of the two drugs³⁰. The results of this trial are almost similar to those of the USA trial and are seen in Table 5. Similar results with those of the former two multicenter trials were obtained and from other studies which evaluated the efficacy of corticosteroids and Sulfasalazine in Crohn's Disease³¹⁻³³. Other studies, though, which evaluated Azathioprine and 6-Mercaptopurine, showed that, contrary to the findings of the USA study, these drugs were efficacious in the management of Crohn's Disease in a dose of 3.5 mg/kg/day. These positive findings were the good response in cases resistent to other drugs, the ability to lower the dose of corticosteroids and the healing of fissures³⁴⁻³⁶. Use of these drugs was efficacious in all forms of the disease (ileitis, colitis, ileocolitis), but the results were evident 3 - 6 months after the initiation of therapy. Long-term administration was necessary to sustain remission, while side effects were observed in 10% of the cases. It is probable then, that the absence of response to Azathioprine in the USA study was due to the short time interval of administration, so that there was not enough time for response to become evident. Recently, 6-Mercaptopurine was tried in children and adolescents with good results and no side effects after long time administration³⁷.

Besides the already mentioned drugs. Metronidazole was evaluated in the treatment of Crohn's Disease following observations of good response in some cases³⁸. Later on it was found in an open study that Metronidazole was effective in the treatment of perineal abcesses, fissures and fistulae in a dose of 20 mg/kg/day³⁹. The same authors observed that the former lesions reccur in a cosiderable percentage when the drug is discontinued⁴⁰ and they suggested long-term administration.

Controlled studies, which followed, proved that Metronidazole is effective in Crohn's Disease in a daily dose of 800 -1500 mg. The drug is effective in cases of colitis and ileocolitis and not in cases of ileitis. Compared to Sulfasalazine, was found to be equally effective^{41,42}.

Almost concurrently with Ulcerative Colitis, 5-ASA was evaluated in Crohn's Disease, as well. The first open study in 1983 showed good results in a dose of 1.5 gr/day for six weeks in cases of ileitis and ileocolitis⁴³. Controlled studies, which followed, confirmed the former results, using a dose of 3 gr/day^{44,45}. The effectiveness of 5-ASA in ileitis, where Sulfasalazine is not effective, is most probably due to the fact that the active 5-ASA is released from Sulfasalazine in the large bowel, throughout a bacterial action, although release of 5-ASA in the terminal ileum has been observed⁴⁶.

The most important, though, effect of 5-ASA in Crohn's Disease, besides its action in acute phases, is the prevention of recurrences, which has not been achieved with Sulfasalazine or other drugs. Recent controlled studies have shown that 5-ASA administered in a dose of 1.5 - 2.4 gr/day in patients who are in remission or other surgery, show less recurrences and, mostly, those with ileitis^{47,48}. The superiority of 5-ASA against Sulfasalazine in the prevention of recurrences is most probably due to the higher amount of active ingredient present in the lumen of the bowel.

In conclusion, it can be said that, in mild and moderate cases of Crohn's Disease, medical treatment is based on the same drugs as in Ulcerative Colitis, with some modifications. These are the higher daily doses of the drugs, the tendency to apply monotherapy and the availability of one more drug that is Metronidazole. Corticosteroids are considered to represent the most effective treatment in all forms of the disease. Sulfasalazine and 5-ASA are indicated in mild cases and so does Metronidazole. while Azathioprine is indicated in resistent to other drugs cases. Local therapy with corticosteroids or 5-ASA suppositories and enemas is indicated in cases of rectal involvement. 5-ASA per os is an effective treatment for the prevention of recurrences.

### INNOVATION

The last five years, I have noticed that 5 of my patients (3 with Ulcerative Colitis and 2 with Crohn's Disease), who experienced frequent recurrences and who were under constant treatment with the traditional drugs, became symptom-free and remained so with no treatment.

I was impressed by this fact and, after investigating these cases, I concluded that the common denominator, which led to remission of their symptoms, was the ingestion of the extract of a herb. The herb is called **Thymus Vulgaris** and its extract was used by these patients for various reasons, such is anorexia, dyspepsia, bronchitis or laryngitis.

I informed 3 more patients of mine (1 with Ulcerative Colitis and 2 with Cronh's Disease) about my observation. These 3 patients suffered from corticosteroid dependent disease and they decided, in their own will, to receive the extract of Thymus Vulgaris. **In all three patients it was possible to taper and discontinue corticosteroids after a month with no recurrence of symptoms.**

On the basis of my observations in these 8 patients with Ulcerative Colitis or Crohn's Disease, I have concluded that in the extract of Thymus Vulgaris there are one or more substances which act therapeutically in these two diseases.

The extract of Thymus Vulgaris has been used for centuries by the people for various symptoms and diseases, such as anorexia, dyspepsia, bronchitis etc. **Until now, though, no drug has been produced from the extract for any disease and it has not been used in the treatment of Ulcerative Colitis and Crohn's Disease.**

As it is known from the bibliography^{49,50,51}, the leaves and the flowers of the herb contain, in a percentage of 1 - 2,5% a volatile oil, which contains various substances. The most important of them are **phenol, thymol, carvacrol, tannin, saponin, terpenic bodies, esters of terpenic bodies and triterpenic acids.**

Based on the properties of these substances, which are contained in the extract of Thymus Vulgaris, I believe that, most probably, the triterpenic acids are the potent ingredient of the extract, given that they posse antinflammatory properties.

**TABLE 1**

| **DRUGS WHICH HAVE BEEN TRIED IN THE TREATMENT OF INFLAMMATORY BOWEL DISEASE** | | | |
|---|---|---|---|
| 1.- | Corticosteroids | 12.- | Hydroxychloroquine |
| 2.- | ACTH | 13.- | 5-ASA |
| 3.- | Sulfasalazine | 14.- | Lidocaine |
| 4.- | Azathioprine | 15.- | Acetarsol |
| 5.- | 6-Mercaptopurine | 16.- | Levamisol |
| 6.- | Cyclosporin | 17.- | Clonidine |
| 7.- | Methotrexate | 18.- | Prostaglandins |
| 8.- | Bismuth Subsalicylate | 19.- | Vancomycin |
| 9.- | Sucralfate | 20.- | Butyrate |
| 10.- | Disodium Cromoglycate | 21.- | Nicotine |
| 11.- | Metronidazole | 22.- | Fish Oil |
| | | 23.- | Newer Corticosteroids |

**TABLE 2**

| **DRUGS WHICH WERE EVALUATED AND WERE FOUND EFFECTIVE IN THE TREATMENT OF INFLAMMATORY BOWEL DISEASE AND THEY ARE IN CURRENT USE** | |
|---|---|
| 1.- | Corticosteroids |
| 2.- | ACTH |
| 3.- | Sulfasalazine |
| 4.- | Azathioprine |
| 5.- | 6-Mercaptopurine |
| 6.- | Metronidazole |
| 7.- | 5-ASA |

**TABLE 3**

| **USUAL THERAPEUTIC PATTERNS IN MILD AND MODERATE CASES OF ULCERATIVE COLITIS** | | | | |
|---|---|---|---|---|
| | | Proctitis | Rectosigmoiditis | Left Colitis Extensive Pancolitis |
| Suppositories: | Prednisolone | + | | |
| | 5-ASA | + | | |
| Enemas: | Hydrocortisone | | + | + |
| | Betamethazone | | | |
| | Budesomide | | | |
| | 5-ASA | | + | + |
| Per os: | Sulfasalazine 5-ASA | + | + | + |
| | Prednisolone | | + | + |

**TABLE 4**

| **CONCLUSIONS OF THE USA MULTICENTER STUDY FOR THE EVALUATION OF PREDNISONE, SULFASALAZINE AND AZATHIOPRINE IN THE TREATMENT OF CROHN'S DISEASE (Gastroenterology 77:847, 1979)** | |
|---|---|
| 1.- | Prednisone and Sulfasalazine are effective agents in the treatment of Crohn's Disease, while Azathioprine is not. |
| 2.- | Prednisone and Sulfasalazine are effective in ilcocolitis. |
| 3.- | Prednisone is more effective in ileitis. |
| 4.- | Sulfasalazine is more effective in colitis. |
| 5.- | If there is no response to either of the two drugs, substitution with the other is not probable to show any effect. |
| 6.- | Sulfasalazine has better results in patients with no previous treatment. |
| 7.- | None of the former drugs can prevent recurrence. |
| 8.- | The simultaneous administration of Prednisone and Sulfasalazine is not superior to Prednisone alone and does not allow lowering of the dose of Prednisone²⁹. |

**TABLE 5**

| **CONCLUSIONS OF THE EUROPEAN MULTICENTER STUDY FOR THE EVALUATION OF 6-METHYLPREDNISOLONE AND SULFASALAZINE IN THE TREATMENT OF CROHN'S DISEASE (Gastroenterology, 86:249, 1984)** | |
|---|---|
| 1.- | Patients with active disease respond to 6-Methylprednisolone, to the combination of the former with Sulfasalazine and to Sulfasalazine. |
| 2. | Patients who respond to treatment with 6-Methylprednisolone, or to combination with Sulfasalazine, remain in remission with small doses of the first or of both. |
| 3.- | Effectiveness of Sulfasalazine was not impressive most probably due to the small dose used. No effect was seen in ileitis. |
| 4. | The combination of 6-Methylprednisolone and Sulfasalazine is not superior to the administration of 6-Methylprednisolone alone. The combination of the two drugs was effective in patients with no prior treatment and in colitis. |
| 5.- | Patients in remission get no benefit from preventive treatment. |

### BIBLIOGRAPHY

1.- Truelove S.C., Witts L.J.: Cortisone in Ulcerative Colitis: Final Report on a therapeutic trial. Br. Med. J. 2:1041, 1955
2.- Truelove S.C., Witts I.J.: Cortisone and Corticotrophin in Ulcerative Colitis. Br. Med. J. 1:387, 1959
3.- Powell-Tuck J., Buckell N.A., Lennard-Jones J.E.: A controlled comparison of Corticotrophin and Hydrocortisone in the treatment of severe Proctocolitis. Scan. J. Gastroenterol. 12:971, 1977
4.- Meyers S., Sachar D.B., Goldberg J. et al: Corticotrophin versus Hydrocortisone in the entravenous treatment of Ulcerative Colitis. A prospective randomized double-blind clinical trial. Gastroenterology 85:351, 1983
5.- Truelove S.C.: Treatment of Ulcerative Colitis with local Hydrocortisone Hemisuccinate Sodium: A report on a controlled therapeutic trial. Br. Med. J. 2:1072, 1952
6.- Baron J.H., Connell A.M., Lennard-Jones J.E. et al: Sulphasalazine and Salicylazosulphadimidine in Ulcerative Colitis. Lancet 1:1094, 1962
7.- Truelove S.C., Watkinson G., Draper G.: Comparison of Corticosteroid and Sulphasalazine therapy in Ulcerative Colitis. Br. Med. J. 2:1708, 1962
8.- Misiewicz J.J., Lennard-Jones J.E., Connell A.M. et al: Controlled trial of Sulphasalazine in maintenance therapy for Ulcerative Colitis. Lancet 1:185, 1965
9.- Moller C., Kiviluoto O., Santavieta S. et al: Local treatment of Ulcerative Proctitis with Salicylazosulphapyridine enema. Clin. Trials J. 15:199, 1978
10.- Jewell P.D., Truelove S.C.: Azathioprine in Ulcerative Colitis. Final Report on controlled therapeutic trial. Br. Med. J. 4:627, 1974
11.- Rosenberg J.L., Wall A.J., Levin B. et al: A controlled trial of Azathioprine in the management of chronic Ulcerative Colitis. Gastroenterology 69:96, 1975
12.- Azad Khan A.K., Piris J., Truelove S.C.: An experiment to determine the active therapeutic moiety of Sulphasalazine. Lancet 2:892, 1977
13.- Klotz U., Maier K., Fisher C. et al: Therapeutic efficacy of Sulfasalazine and its metabolites in patients with Ulcerative Colitis and Chron's Disease. N. Engl. J. Med. 303:1499, 1980
14.- Campieri M., Lanfranchi G.A., Bazzocchi G. et al: Treatment of Ulcerative Colitis with high dose 5-Aminosalicylic acid enemas. Lancet 2:270, 1981
15.- Maier K., Frughnorgen P., Bode J.C. et al: Successful management of chronic Inflammatory Bowel Disease with oral 5-Aminosalicylic acid. Dtsch. Med. Wochensehr 110:363, 1985
16.- Meyers S., Sachaz D.B., Present D.H., Janowitz H.D.: Olsalazine Sodium in the treatment of Ulcerative Colitis among patients intolerant of Sulfasalazine. Gastroenterology 93:1255, 1987
17.- Powell-Tuck J., Brown R.L., Lennard-Jones J.E.: A comparison of oral Prednisone given as a single or multiple daily doses for active Proctocolitis. Scand. J. Gastroenterol. 13:883, 1978
18.- Jewell D.: Inflammatory Bowel Disease. Medicine 61:615, 1982
19.- Willliams C.N. : Clinical experience with 5-Aminosalicylate preparations in Inflammatory Bowel Disease: A review. Can. J. Gastroenterol. 1:28, 1987
20.- Present D.H.: The role of Immunosuppressives in Inflammatory Bowel Disease: In recent developments in the therapy of Inflammatory Bowel Disease. Proceedings of a symposium. Baltimore 1986
21.- Lennard-Jones J.E., Misiewicz J.J., Connell A.M. et al: Prednisone as maintenance treatment for Ulcerative Colitis in remission. Lancet 1:188, 1965
22.- Azad Khan A.K., Howes D.T., Piris J. et al: Optimum dose of Sulphasalazine for maintenance treatment of Ulcerative Colitis. Gut. 21:232, 1980
23.- Biddle W.L., Greenberger N.J., Swan J.T. et al: 5-Aminosalicylic acid enemas: Effective agent in maintaining remission in left sided Ulcerative Colitis. Gastroenterology 94:1075, 1988
24.- Riley S.A., Mani V., Goodman M.J. et al: Comparison of delayed-release 5-Aminosalicylic acid (Mesalasine) and Sulfasalazine as maintenance treatment for patients with Ulcerative Colitis. Gastroenterology 94:1383, 1988
25.- Mulder C.J.J., Tytgat G.N.J., Waterman I.T. et al: Double-blind comparison of slow-release 5-Aminosalicylate and Sulfasalazine in remission maintenance in Ulcerative Colitis. Gastroenterology 95:1449, 1988
26.- Dissanayake A.S., Truelove S.C.: A controlled trial of long-term maintenance treatment of Ulcerative Colitis with Sulphasalazine (Salazopyrin), Gut. 14:923, 1974
27.- Riis P., Anthonisen P., Wulff H.A. et al: The prophylactic effect of Salazosulphapyridine in Ulcerative Colitis during long-term treatment. Scand. J. 8:71, 1973
28.- Summers R.W., Switz D.M., Sessions J.T. Jr et al: National Cooperative Crohn's Disease Study: Results of Drug Treatment. Gastroenterology 77:847, 1979
29.- Singleton J.W., Summers R.W., Kern F. Jr et al: A trial of Sulfasalazine as adjunctive therapy in Crohn's Disease. Gastroenterology 77:887, 1979
30.- Malchow H., Ewe K., Brandes J.W. et al: European Cooperative Crohn's Disease Study: Results of Drug Treatment. Gastroenterology 86:249, 1984
31.- Cooke W.T., Fielding J.F.: Corticosteroid or Corticotrophin therapy in Crohn's Disease. Gut. 11:921, 1970
32.- Anthonisen P., Barany F., Fokenborg O. et al: The clinical effect of Salazosulphapyridine (Salazopyrin) in Crohn's Disease. Scand. J. Gastroenterol. 9:549, 1974
33.- Van Hees P.A.M., Van Lier H.J.J., Van Elteren P.H. et al: Effect of Sulfasalazine in patients with active Crohn's Disease. A controlled double-blind study. Gut. 22:404, 1981
34.- Willoughhy J.M.T., Kumar P.J., Beckett J. et al; Controlled trial of Azathioprine in Crohn's Disease. Lancet 2:944, 1971
35.- Present D.H., Korelitz B.I., Wisch N. et al: Treatment of Crohn's Disease with 6-Mercaptopurine. A long-term randomized, double-blind study. N. Engl. J. Med. 302:981, 1980
36.- O'Brien J.J., Bayless T.M., Bayless J.A.: Use of Azathioprine or 6-Mercaptopurine in the treatment of Crohn's Disease. Gastroenterology 101:39. 1991
37.- Markowitz J., Rosa J., Grancher K. et al: Long-term 6-Mercaptopurine treatment in adolescents with Crohn's Disease. Gastroenterology 99:1347, 1990
38.- Ursing B., Kamme C.: Metronidazole for Crohn's Disease. Lancet 1:755, 1975
39.- Bernstein L.H., Frank M.S., Brandt L.J.: Healing of perineal Crohn's Disease with Metronidazole. Gastroenterology 79:357, 1980
40.- Brandt M.S., Bernstein L.H., Boley S.J., Frank M.: Long-term follow-up of Metronidazole for perineal Crohn's Disease. Gastroenterology 80:1115 (Abstr.), 1981
41.- Ursing B., Alm T., Barany F. et al: A comparative study of Metronidazole and Sulfasalazine for active Crohn's Disease. The Cooperative Disease Study in Sweden. II Result. Gastroenterology 83:550, 1982
42.- Sutherland L., Singleton J., Sessions J. et al; Metronidazole in Crohn's Disease: What's the score? Gut. 32:1071, 1991
43.- Rasmussen S.N., Binder V., Maier K. et al: Treatment of Crohn's Disease with peroral 5-Aminosalicylic acid. Gastroenterology 85:1350, 1983
44.- Rasmussen S.N., Lauritsen K., Tagejensen U. et al: 5-Aminosalicylic acid in the treatment of Crohn's Disease. A 16-week double-blind, controlled, multicentre study with Pentasa. Scand. J. Gastroenterol. 22:877, 1987
45.- Maier K., Frick H., Gaisberg U. et al: Clinical efficacy of oral Mesalazine in Crohn's Disease. Can. J. Gastroenterol. 4:13, 1990
46.- Peppercorn M.A.: Advances ih the drug therapy for Inflammatory Bowel Disease. Ann. Intern. Med. 112:50, 1990
47.- Thomson A.B.R.: International Mesalazine study group. Coated oral 5-Aminosalicylic acid vs placebo in maintaining remission of inactive Crohn's Disease. Aliment. Pharmacol. Therap. 4:55, 1990
48.- Prantera C., Pallone F., Brunetti G. et al: Oral 5-ASA in the maintenance treatment of Crohn's Disease. Results of a randomized placebo controlled trial with Asacol. Gastroenterology 103:363, 1992
49.- Schauenberg P., Paris F.: Guide of the pharmaceutical plants. Ed. M. Ghiourdas, Athens, 1981
50.- Fokas G.: Lectures of pharmacopoeia. Thessaloniki, 1979
51.- Mabey R.: The complete new herbal. Gaia Books Ltd., London, 1991

## Claims

1. Use of the extract of Thymus Vulgaris, alone or in combination, for the preparation of a medicament for the treatment of Ulccrative Colitis and Crohn's Disease.

2. Use of Triterpenic acids, alone or in combination, for the preparation of a medicament for the treatment of Ulcerative Colitis and Crohn's Disease.

3. Use of Phenol, alone or in combination, for the preparation of a medicament for the treatment of Ulcerative Colitis and Crohn's Disease.

4. Use of Thymol, alone or in combination, for the preparation of a medicament for the treatment of Ulcerative Colitis and Crohn's Disease.

5. Use of Carvacrol, alone or in combination, for the preparation of a medicament for the treatment of Ulcerative Colitis and Crohn's Disease.

6. Use of Tannin, alone or in combination, for the preparation of a medicament for the treatment of Ulcerative Colitis and Crohn's Disease.

7. Use of Saponin, alone or in combination, for the preparation of a medicament for the treatment of Ulcerative Colitis and Crohn's Disease.

8. Use of Terpenic bodies, alone or in combination, for the preparation of a medicament for the treatment of Ulcerative Colitis and Crohn's Disease.

9. Use of Esters of Terpenic bodies, alone or in combination, for the preparation of a medicament for the treatment of Ulcerative Colitis and Crohn's Disease.
